(19) Europäisches Patentamt
European Patent Office
Office européen des brevets

(11) **EP 3 884 250 B1**

(12) **EUROPEAN PATENT SPECIFICATION**

(45) Date of publication and mention
of the grant of the patent:
**08.06.2022 Bulletin 2022/23**

(21) Application number: **19809577.0**

(22) Date of filing: **15.11.2019**

(51) International Patent Classification (IPC):
*G01K 7/02* (2021.01)

(52) Cooperative Patent Classification (CPC):
**G01K 7/02; G01K 17/00;** G01N 33/15

(86) International application number:
**PCT/GB2019/053254**

(87) International publication number:
**WO 2020/104774 (28.05.2020 Gazette 2020/22)**

(54) **APPARATUS AND METHODS FOR THERMALLY TESTING A SAMPLE**

VORRICHTUNG UND VERFAHREN ZUM THERMISCHEN TESTEN EINER PROBE

APPAREIL ET PROCÉDÉS POUR TESTER THERMIQUEMENT UN ÉCHANTILLON

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.11.2018 GB 201818888**

(43) Date of publication of application:
**29.09.2021 Bulletin 2021/39**

(73) Proprietor: **Oxford University Innovation Limited
Oxford, Oxfordshire OX2 0JB (GB)**

(72) Inventors:
• **CHANA, Kamaljit Singh
Oxford, Oxfordshire OX2 0ES (GB)**
• **SRIDHAR, Vikram
Oxford, Oxfordshire OX2 0ES (GB)**
• **KHIMJI, Saleema
Toronto, Ontario M5J 0B5 (CA)**

(74) Representative: **J A Kemp LLP
80 Turnmill Street
London EC1M 5QU (GB)**

(56) References cited:
**CN-A- 107 271 481     RU-C1- 2 273 005**

**Description**

The present invention relates to testing samples, particularly pharmaceutical products for the purpose of detecting counterfeit medication in a low cost and/or portable device.

The World Health Organisation (WHO) currently estimates that 1 in 10 medical products in low- and middle-income countries is substandard or falsified. In a 2017 WHO study involving more than 48,000 medicines, 65% of fake medicines were found to involve drugs for treating malaria and bacterial infections. Every year, more than 122,000 African children under the age of five lose their lives as a result of counterfeit antimalarials alone. Both generic and innovator medicines can be falsified, ranging from very expensive products for cancer to very inexpensive products for treatment of pain.

Currently a suspected drug would have its package analysed visually to see whether it is a fake. Counterfeit manufacturers are making that job much more difficult. Alternative procedures may use one or more of the following: chemical analysis; chromatography; spectroscopy; chemical induced colour-based testing; hardness and dissolutions testing; infrared & near infrared testing; Raman Spectroscopy; ultraviolet testing; x-ray diffraction (XRD); and x-ray fluorescence (XRF). These alternative procedures are generally costly and/or time consuming and may not be practical at the point of sale, particularly in low- and middle-income countries.

CN 107 271 481 A discloses a multi-directional measuring apparatus and method for the thermal conductivity of soil. The multi-directional measuring apparatus comprises temperature sensor probes, a probe seat, a heat pulse probe, internal thread mouths, I-shaped bosses, I-shaped chutes, thermocouples, a heat pulse generator, a hand-held controller and a base plate.

RU 2 273 005 C1 discloses methods and devices for measuring the temperature of a heating spiral. The heating spiral is connected into the break of connection of thermal wires of a thermocouple, which thermal wires are capable of forming a working junction. Values of thermoelectromotive force of the thermocouple are registered and the temperature of the heating spiral is calculated from a difference in temperatures of the warmed and cold ends of the thermocouple.

It is an object of the invention to at least partially address one or more of the above problems.

According to an aspect, there is provided an apparatus for testing a sample, comprising: a sensor element comprising a first conductor, a second conductor and a third conductor, wherein: the first conductor, second conductor and third conductor are connected together electrically in series; the first conductor is connected to the second conductor at a first sensing junction; the second conductor is connected to the third conductor at a second sensing junction; and the apparatus further comprises a measurement unit configured to: apply heating to a sample under test via Joule heating in the second conductor, the Joule heating being generated by driving an electrical current in series through the first conductor, second conductor and third conductor; measure a thermal response of the sample to the heating by measuring a potential difference between the first conductor and the third conductor, wherein the potential difference is influenced by a temperature gradient along the first conductor and a temperature gradient along the third conductor via the Seebeck effect; and generate an indication of whether the sample satisfies a predetermined criterion based on the measured thermal response.

Thus, an apparatus is provided that can be manufactured using reliable and rugged components at low cost. Components that have been mass produced for traditional thermocouple devices may be used to provide the first and third conductors and some of the electronics used to measure temperature in the region of the first and second sensing junctions. In comparison with a traditional thermocouple device, the second conductor effectively provides an extended junction region between components (the first and third conductors) that perform a role similar to the positive and negative legs of the thermocouple device. However, the second conductor provides the additional possibility of applying heating directly in the location where the thermal response is to be measured without requiring separate electrical connections and a separate resistive heating element. Errors associated with differences in location between the heating and the measurement of the thermal response are avoided. The averaging effect on the measurement of temperature provided by the extended junction region further reduces errors and reduces the risk of anomalies due to local variations or spikes in temperature. The robustness of the sensor element, in comparison with alternative approaches based on thin film measurements for example, also means that the heating can be applied for a relatively long period of time. This allows compositional properties to be sampled to a greater depth in the case where the sample (e.g. pharmaceutical product) is solid, which may be used to reduce errors by further spatial averaging or to detect anomalies beneath the surface of the sample.

The measurement unit can be implemented based on electronics for obtaining a temperature measurement from a standard thermocouple, which is widely available and low cost. Adaptation to provide the heating capability can also be implemented at low cost. Combining the above advantages with the relatively low power requirements means that the apparatus can easily be implemented in portable form, for example in a hand-held device. The apparatus is thus suitable for use in the field, such as in pharmacies, shops, or health facilities in remote locations, and even in a domestic context.

In an embodiment, the electrical resistance of the second conductor is at least 2 times higher than the electrical resistance of the first conductor and than the electrical resistance of the third conductor. The Joule heating thus occurs predominantly in the second conductor. This minimizes overall power requirements and helps to focus heating to the region being tested, which improves accuracy.

In an embodiment, the second conductor is locally elongate and has a length that is at least 2 times longer than a shortest distance between the first sensing junction and the second sensing junction. Thus, high resistance can be achieved without a cross-sectional area of the second conductor needing to become excessively small (which might reduce ruggedness and/or increase cost of manufacture by requiring manufacturing steps having higher levels of precision). Additionally, the spatial separation between the first and second sensing junctions can be kept relatively small, which ensures that the measurements based on the Seebeck effect accurately reflect the thermal response of the region between the first and second sensing junctions.

In an embodiment, at least part of the first conductor, second conductor, and third conductor are embedded in a matrix material; and one or more of the following are flush with an outer surface of the matrix material and can be brought into direct contact with the sample under test: the first sensing junction, the second sensing junction, the second conductive material. This arrangement can be manufactured efficiently by embedding the components in the material matrix and machining (e.g. lapping) the matrix material until the components are made flush with the outer surface. The components flush with the outer surface can be brought into good thermal contact with the sample to be tested, thereby providing good sensitivity, while at the same time being maximally supported by the matrix material, which enhances robustness, longevity and reliability.

According to another aspect, there is provided a method of testing a sample, comprising: providing a sensor element comprising a first conductor, a second conductor and a third conductor, wherein: the first conductor, second conductor and third conductor are connected together electrically in series; the first conductor is connected to the second conductor at a first sensing junction; the second conductor is connected to the third conductor at a second sensing junction; and the method further comprises: applying heating to a sample under test via Joule heating in the second conductor, the Joule heating being generated by driving an electrical current in series through the first conductor, second conductor and third conductor; measuring a thermal response of the sample to the heating by measuring a potential difference between the first conductor and the third conductor, wherein the potential difference is influenced by a temperature gradient along the first conductor and a temperature gradient along the third conductor via the Seebeck effect; and generating an indication of whether the sample satisfies a predetermined criterion based on the measured thermal response.

Embodiments of the invention will now be described, by way of example only, with reference to the accompanying drawings in which corresponding reference symbols indicate corresponding parts, and in which:

Figure 1 schematically depicts an example apparatus for testing a pharmaceutical product as sample;
Figure 2 is a magnified view of a probe tip of the apparatus of claim 1;
Figure 3 schematically depicts a thermocouple device;
Figure 4 schematically depicts a sensor element of the apparatus of Figure 1;
Figure 5 is a graph schematically depicting application of a heating pulse and a variation in temperature with time measured by the sensor element;
Figure 6 is a schematic end view of a sensor element showing a serpentine shaped second conductor;
Figure 7 is a schematic side sectional view of a probe tip before lapping away of a matrix material during manufacture;
Figure 8 is a schematic side sectional view of the probe tip of Figure 7 after lapping away of the matrix material to make first and second sensing junctions flush with an outer surface of the matrix material;
Figures 9 and 10 are graphs depicting experimental results using an apparatus of the type depicted in Figure 1 for different materials; and
Figure 11 depicts testing of a sample via sensor elements engaging with opposite sides of the sample.

Embodiments of the present disclosure provide apparatus and methods for testing a sample, for example a pharmaceutical product, to determine whether the sample satisfies a predetermined criterion. The predetermined criterion may relate to whether a pharmaceutical product is a genuine version of the pharmaceutical product, whether the pharmaceutical product has reached a predetermined level of quality or purity, and/or whether the pharmaceutical product contains less than a desired minimum amount of a target substance or more than a desired maximum amount of an undesired impurity. The testing procedure may thus comprise determining compositional information about a pharmaceutical product. The compositional information may comprise any compositional property that affects thermal characteristics, in particular heat transfer characteristics, of the pharmaceutical product. The compositional information may comprise chemical or structural information. The sample may comprise a solid, for example in the form of a tablet or powder. In an embodiment, the sample comprises a solid body for oral administration (e.g. a tablet), the solid body comprising a pharmaceutically active ingredient. In other embodiments, the sample comprises a liquid, such as a medicine in liquid form.

The methods use a sensor element to apply heating to the sample (e.g. pharmaceutical product). A thermal response (which may be referred to also as a temperature response) of the sample is measured. The thermal response is dependent on a heat transfer characteristic of the sample. The heat transfer characteristic depends on chemical and/or structural properties of the sample. The measured thermal response therefore provides information about chemical and/or structural properties of the sample. The heat transfer characteristic affects how efficiently heat will be conducted away from the sensor element. Applied heat penetrates underneath the surface of the sample being sensed, allowing subsurface structure to be sensed, such as different layers of a pharmaceutical product, or inhomogeneities or inclusions within a pharmaceutical product. The methodology is thus sensitive to pharmaceutical products in which an active or other component is distributed within a matrix material having a different composition. The ability to detect sub-structure makes it possible for the sensing to be performed through materials separating the sensor element from the material of interest, including not only outer layers of a pharmaceutical product but also packaging or other materials that may be present around the pharmaceutical product.

Sensing can be achieved effectively even for relatively low heating power. The method can be performed for example without increasing the local temperature of the sample by more than a few degrees Celsius (e.g. 2-5 degrees Celsius). Thermal damage to the sample is therefore avoided. Overall power requirements are also kept low, facilitating implementation as a portable (e.g. battery powered) device.

Heat transfer characteristics of materials (e.g. thermal properties such as thermal conductivity, $\kappa$, specific heat capacity, c, and quantities that depend on one or both of these properties) can depend sensitively on the composition (e.g. chemical or structural) of the materials. The thermal product, $\sqrt{\rho c \kappa}$, where $\rho$ is equal to the density, is often a heat transfer characteristic that is particularly sensitive to composition because it takes into account both $\kappa$ and c. Changes in either or both of $\kappa$ and c will typically result in a change in $\sqrt{\rho c \kappa}$. Changes in relative concentrations of different components in a multi-component material can be detected where the different components have different thermal properties. Changes in structure can be detected where there is a density or compositional change.

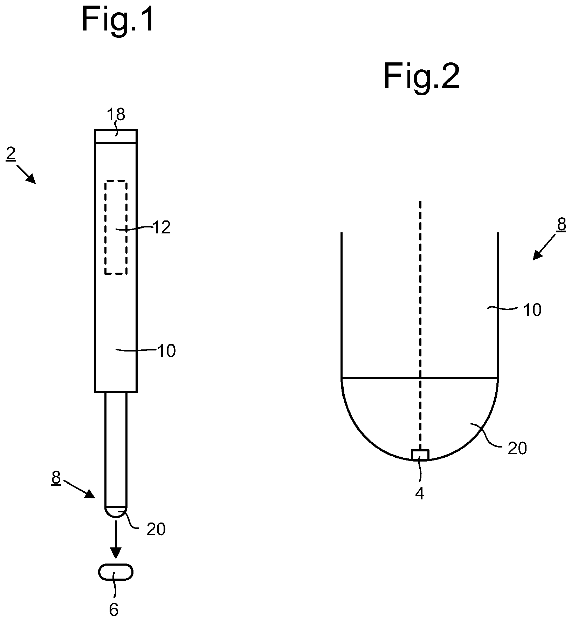

Figures 1 and 2 depict an example apparatus 2 for testing a sample 6, such as a pharmaceutical product. The apparatus 2 comprises a sensor element 4 configured to be brought into thermal contact with the sample (e.g. in direct contact with a surface of the sample or in contact via a thin coupling layer or gel not forming an integral part of the sample 6). In the embodiment shown, the sensor element 4 is provided at the distal end of a probe tip 8. The apparatus may comprise a body 10 that a user can grasp to bring the sensor element 4 into thermal contact with the sample 6. The apparatus 2 may have a shape and/or size that is similar to a writing pen, for example. Where the sample 6 is a liquid, the probe tip 8 may be immersed in the liquid to test the liquid.

The apparatus 2 comprises a measurement unit 12. The measurement unit 12 applies heating to the sample 6 via the sensor element 4. The measurement unit 12 measures a thermal response to the heating to determine compositional information about the sample 6. The thermal response is dependent on a heat transfer characteristic of the sample 6, as discussed above. The measurement unit 12 uses the measured thermal response to provide an indication about whether the sample 6 satisfies a predetermined criterion.

In an embodiment, the probe tip 8 comprises a deformable coupling member 20 that deforms on engagement with the sample 6 when the probe tip 8 is brought into contact with the sample 6. The sensor element 4 is mounted on, in, and/or in thermal contact with, the deformable coupling member 20. The deformable coupling member 20 may be configured to deform elastically (e.g. such that the deformable coupling member 20 is resilient and springs back to an equilibrium shape when the contact between the probe 8 and the sample 6 is removed). In an embodiment, the deformable coupling member 20 comprises a foam material or a deformable membrane (pocket) comprising a fluid such as air. An example arrangement is shown in Figure 2, where the sensor element 4 is provided on or near a distal surface of the deformable coupling member 20. The broken line indicates schematically an electrical connection path from the sensor element 4 towards the measurement unit 12. The electrical connection path may pass through or around the deformable coupling member 20 depending on the nature of the deformable coupling member 20. Where the deformable coupling member 20 comprises a membrane containing a fluid such as air, the electrical connection path may comprise metallic tracks formed on a surface of the membrane.

The deformable coupling member 20 helps a user to maintain a suitable force between the probe 8 and the sample 6 during testing, thereby improving accuracy and repeatability of the measurements.

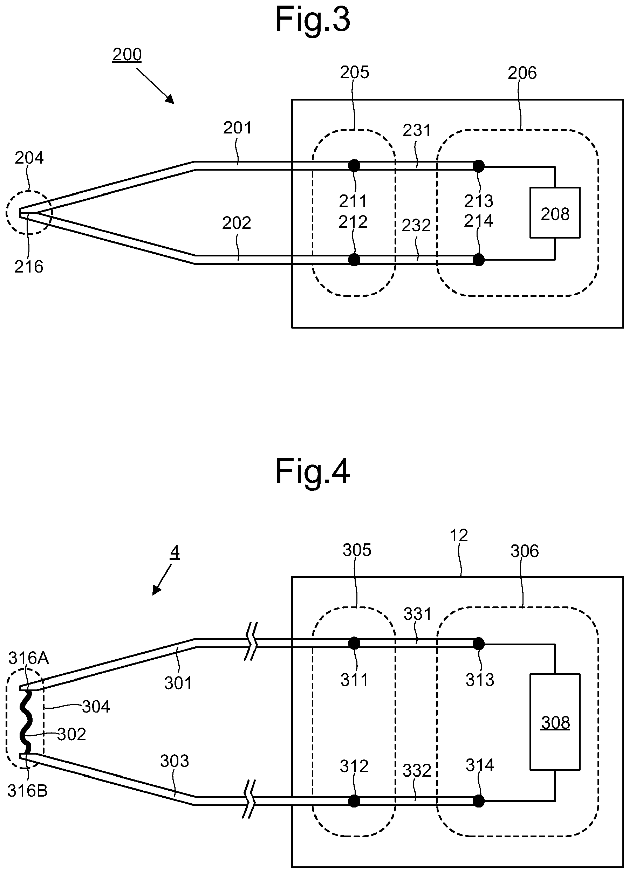

The principle of operation of the sensor element 4 builds on that of traditional thermocouples, so a short discussion of this is now given with reference to Figure 3.

Figure 3 depicts a common measurement configuration for a thermocouple device 200. The thermocouple device 200 comprises a first leg 201 and a second leg 202. The first leg 201 and the second leg 202 are electrically connected together, for example by welding, to form a sensing junction 216. The first leg 201 and the second leg 202 are conductors (e.g. wires) formed from different materials. The first leg 201 is connected at junction 211 to a first measurement wire 231. The second leg 202 is connected at junction 212 to a second measurement wire 232. The first and second measurement wires 231 and 232 are connected at junctions 213 and 214 to a measurement circuit 208. The measurement circuit 208 measures a potential difference between the junctions 213 and 214 in order to determine a temperature $T_s$ at the sensing junction 216.

The potential difference indicative of the tem-

perature $T_s$ at the sensing junction 216 arises because of the Seebeck effect. In a K-type thermocouple, for example, the first leg 201 is formed from chromel and the second leg 202 is formed from alumel. The first and second measurement wires 231 and 232 are formed from the same material as each other (e.g. copper). The temperature in region 205 (containing junctions 211 and 212) is known and may be denoted $T_0$. The temperature in region 204 (containing the sensing junction 216) is then obtained as follows.

The Seebeck effect describes the electromotive force that arises when there is a temperature gradient in an electrically conductive material. When there is no internal current flow, the gradient of the voltage, $\Delta V$, is related to the gradient in temperature, $\Delta T$, by the Seebeck coefficient $S(T)$, which is a temperature dependent material property, as follows:

$$\Delta V = -S(T)\Delta T$$

The potential difference between 213 and 214 does not depend on the temperature in region 206 because the first and second measurement wires 231 and 232 are formed from the same material and have the same temperature gradient along them. The potential difference depends entirely on the properties of the first and second legs 201 and 202 and the temperature difference between $T_0$ and $T_s$. The potential difference is given by the following expression:

$$\int_{T_0}^{T_s} \big(S_+(T) - S_-(T)\big)dT$$

where $S_+$ and $S_-$ are the Seebeck coefficients of the materials forming the first and second legs 201 and 202 (chromel and alumel in the example above). A characteristic function $E(T)$ for a given thermocouple can be established using calibration measurements and the potential difference V between 213 and 214 will be given by the following expression:

$$V = E(T_s) - E(T_0)$$

where

$$E(T) = \int (S_+(T') - S_-(T'))dT' + constant$$

Figure 4 depicts a sensor element 4 and a measurement unit 12 suitable for use with the apparatus of Figure 1. The sensor element 4 comprises a first conductor 301 (e.g. a wire), a second conductor 302 (e.g. a wire) and a third conductor 303 (e.g. a wire). The first conductor 301, second conductor 302 and third conductor 303 are connected together in series between junctions 313 and 314 connected to measurement circuit 308. The measurement circuit 308 may be implemented to provide at least the functionality provided by the measurement circuit 208 used for the thermocouple device 200 of Figure 3 (namely, to measure a potential difference between junctions 313 and 314 caused by the Seebeck effect).

The first conductor 301 is connected to the second conductor 302 (e.g. by welding) at a first sensing junction 316A. The second conductor 302 is connected to the third conductor 303 (e.g. by welding) at a second sensing junction 316B.

The measurement unit 12 applies heating to a sample (e.g. pharmaceutical product) under test provided in region 304 via Joule heating in the second conductor 302. The Joule heating is generated by the measurement circuit 308 driving an electrical current through the series of first conductor 301, second conductor 302 and third conductor 303. The electrical current also flows through first and second measurement wires 331 and 332 respectively connecting the first conductor 301 and the third conductor 303 to the measurement circuit 308.

As in the thermocouple device 200 of Figure 3, the first and second measurement wires 331 and 332 are formed from the same material (e.g. copper). The first measurement wire 331 is connected to the first conductor 301 at junction 311. The second measurement wire 332 is connected to the third conductor 303 at junction 312. Region 305 containing the junctions 311 and 312 is held at a common temperature (i.e. junction 311 is always at the same temperature as junction 312). This may be achieved for example by arranging for the junctions 311 and 312 to be in good thermal contact with each other and/or with a block of material having high thermal conductivity and/or high heat capacity. The region 306 containing junctions 313 and 314 may typically be thermally isolated from the region 305 to avoid heat generated in the measurement circuit 308 perturbing the temperature in the region 305.

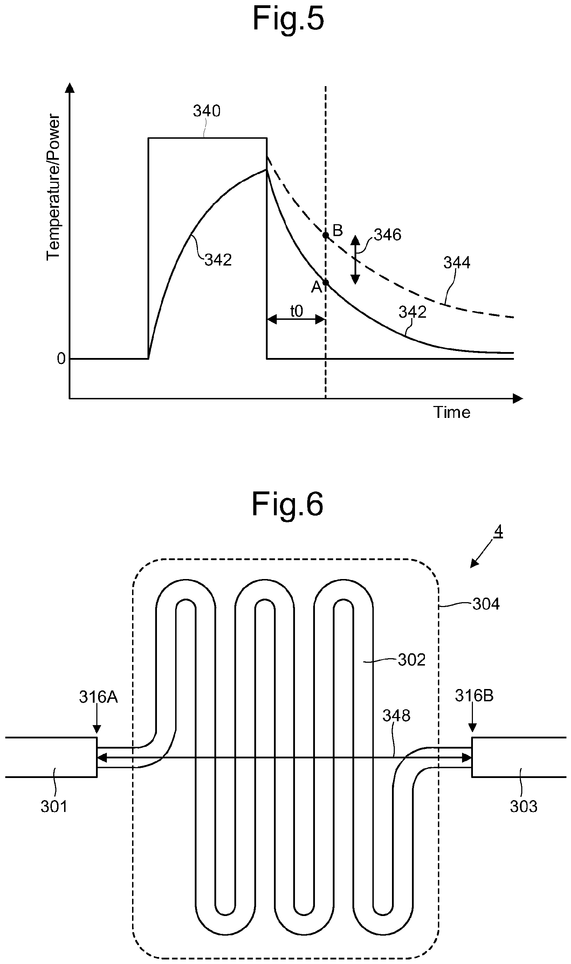

The measurement circuit 308 measures a temperature in the region 304 in thermal contact with the second conductor 302 and thereby measures a thermal response of the sample 6 (which is also in thermal contact with the region 304, for example by being in direct contact with the second conductor 302). The measurement of the thermal response can be performed during or after the application of the Joule heating via the second conductor 302. In the example discussed below with reference to Figure 5, the heating is applied before the measurement of the thermal response. Thus, the heating of the sample 6 and the measurement of the thermal response are performed in non-overlapping time periods.

The measurement of the thermal response is performed by measuring a potential difference between the first conductor 301 and the third conductor 303. The potential difference is influenced by the Seebeck effect associated with a temperature gradient along the first conductor 301 and a temperature gradient along the third conductor 303. In the example of Figure 4, the temperature gradient along the first conductor 301 is between the temperature in region 305 and the temperature at the first sensing junction 316A. The temperature gradient along the third conductor 303 is between the temperature in region 305 and the temperature at the second sensing junction 316B.

The first conductor 301 consists of a first material. The third conductor 303 consists of a second material. The first material and the second material are different, such that a potential difference generated by the temperature gradient along the first conductor 301 is different to the potential difference generated by the temperature gradient along the third conductor 303. In an embodiment, the first and second materials are selected to match pairs of materials used for the positive and negative legs of traditional thermocouple devices such as the thermocouple device 200 discussed above with reference to Figure 3. For example, the first material and the third material may respectively comprise, consist essentially of, or consist of one of the following pairs of materials: chromel and constantan as for a type E thermocouple device; iron and constantan as for a type J thermocouple device; chromel and alumel as for a type K thermocouple device; 82%Ni/18%Mo and 99.2%Ni/0.8%Co, by weight, as for a type M thermocouple device; Nicrosil and Nisil as for a type N thermocouple device; copper and constantan as for a type T thermocouple; 70%Pt/30%Rh and 94%Pt/6%Rh, by weight, as for a type B thermocouple device; 87%Pt/13%Rh by weight and platinum as for a type R thermocouple device; 90%Pt/10%Rh by weight and platinum as for a type S thermocouple device; 95%W/5%Re and 74%W/26%Re, by weight, as for a type C thermocouple device; 97%W/3%Re and 75%W/25%Re, by weight, as for a type D thermocouple device; and Tungsten and 74%W/26%Re by weight as for a type G thermocouple device.

In an embodiment, as depicted in Figure 5, the heating of the sample 6 is performed by driving a constant current through the second conductor 302 for a predetermined period of time. A square wave heating pulse 340 is thus applied to the sample 6 (in thermal contact with region 304). The curve 342 schematically depicts how the temperature of the sample 6 is expected to change as a function of time. The temperature increases during the heating pulse 340 and decreases outside of the heating pulse 340. The measurement of the temperature by the sensor element 4 is performed outside of the heating pulse 340. This is an example of a class of embodiments in which the heating and the measurement of the thermal response are performed in non-overlapping time periods. Performing the heating and the measurement of the thermal response in non-overlapping time periods simplifies the electronics required to implement the measurement unit 12 significantly.

In an embodiment, a response to the heating pulse is compared with the response to a corresponding heating pulse applied to a reference material. The size of the response, the variation of the response as a function of time, or various other aspects of the response may be considered. Any deviation from the response measured for the reference material may be used to detect a deviation from normality for the sample 6 being tested. The nature of the heating pulses may be selected to achieve optimum sensitivity for the particular type of sample (e.g. pharmaceutical product) being tested. This may involve selecting particular pulse shapes, amplitudes, durations and/or repetition rates, for example.

The measurement unit 12 generates an indication of whether the sample 6 satisfies a predetermined criterion based on the measured thermal response. In an embodiment, this is achieved by comparing the measured thermal response to a corresponding measured response obtained at an earlier time for a reference sample (e.g. a reference pharmaceutical product). In a case where the apparatus 2 is being used to detect counterfeit drugs, for example, the reference pharmaceutical product 6 may consist of a genuine version of the drug in question. In one implementation, the apparatus 2 is provided with a user input unit 18 (e.g. a button), as exemplified schematically in Figure 1. A user positions the probe tip 8 so that the sensor element 4 is in thermal contact with a reference pharmaceutical product (e.g. a product known to be of acceptable quality and/or authentic). The user provides an input to the apparatus 2 via the user input unit 18 (e.g. by pressing a button) to cause the apparatus 2 to measure the reference pharmaceutical product by applying a heating pulse to the sensor element 4. The thermal response of the reference pharmaceutical product is stored. The user then repositions the probe tip 8 so that the sensor element 4 is in thermal contact with a pharmaceutical product 6 to be tested. The user provides a further input to the apparatus 2 via the user input unit 18 (e.g. by pressing a button) to cause the apparatus 2 to measure the pharmaceutical product 6 to be tested. The measurement unit 12 compares the measured thermal response from the pharmaceutical product 6 to be tested with the measured thermal response from the reference pharmaceutical product.

In the example of Figure 5, the measurement unit 12 measures a temperature of the sample 6 at a time point corresponding to a predetermined period of time t0 after the end of the heating pulse 340. An expected variation of temperature with time for the reference sample is indicated by curve 344. The measured temperature at point A is then compared with the expected measured temperature at point B on the curve 344 for the reference sample. If the deviation 346 is less than a first threshold value the measurement unit 12 generates an indication to show that the sample 6 has "passed" the test (and is therefore of sufficiently high quality, where the testing is being done as part of a quality control procedure, or is authentic, wherein the testing is being done to detect fake versions of the pharmaceutical product). This may be indicated for example by illuminating a green LED or by providing a suitable indication or text on a display (not shown). If the deviation 346 is more than a second threshold value but less than a third threshold value, the measurement unit 12 generates an indication to show that the sample 6 is "suspect" and that further investigation may be required. This may be indicated for example by illuminating an orange LED or by providing a suitable indication or text on a display. If the deviation 346 is more than the third threshold value the measurement unit 12 generates an indication to show that the sample 6 has "failed" the test (e.g. is deemed to be a counterfeit version of the drug). This may be indicated for example by illuminating a red LED or by providing a suitable indication or text on a display.

In an embodiment, a majority of the electrical resistance in the circuit through which current is driven to provide the Joule heating is contributed by the second conductor 302. In an embodiment, the electrical resistance of the second conductor 302 is at least 2 times, optionally at least 5 times, optionally at least 10 times, higher than the electrical resistance of the first conductor 301 and than the electrical resistance of the third conductor 303. The Joule heating is thus focussed spatially in the region of the second conductor 302. In an embodiment, the separation between the first and second sensing junctions 316A and 316B is less than the length of either of the first and third conductors 301 and 303. Thus, not only is the overall amount of Joule heating occurring in the second conductor 302 higher than in either of the first and third conductors 301 and 303, the heating is concentrated spatially, leading to a majority of the power per unit volume being delivered in the region 304.

In an embodiment, an average cross-sectional area (e.g. averaged longitudinally) of the second conductor 302 is at least 2 times, optionally at least 5 times, optionally at least 10 times, smaller than an average cross-sectional area (e.g. averaged longitudinally) of the first conductor 301 and than an average cross-sectional area (e.g. averaged longitudinally) of the third conductor 303. This approach helps to achieve high resistance without excessively increasing the length of the second conductor 302.

Alternatively or additionally, as depicted in Figure 6, the second conductor 302 is locally elongate (e.g. like a wire) and has a length that is at least 2 times, optionally at least 5 times, optionally at least 10 times, longer than a shortest distance 348 between the first sensing junction 316A and the second sensing junction 316B. For example, the conductor 302 may adopt a sinuous form, such as a spiral, a helix or a serpentine shape. An example of a serpentine geometry is depicted in Figure 6.

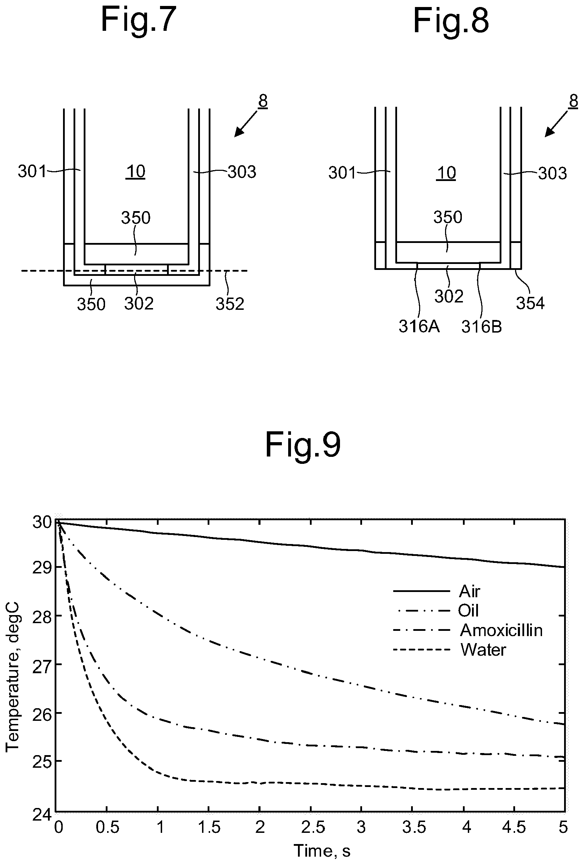

Figures 7 and 8 depict a probe tip 8 at different stages of manufacture of an example apparatus 2 for testing a sample. In Figure 7 a distal part of the first conductor 301, the second conductor 302, and a distal part of the third conductor 303 are all embedded in a matrix material 350. Various materials may be used for the matrix material. In an embodiment, the matrix material comprises an acrylic. In a subsequent processing step, the probe tip 8 is lapped down to a target plane 352, which exposes the first and second sensing junctions 316A and 316B, as depicted in Figure 8. The embodiment of Figure 8 is thus an example of a class of embodiments in which each of at least part of the first conductor 301, at least part of the second conductor 302, and at least part of the third conductor 303 is embedded in a matrix material 350. The first sensing junction 316A, second sensing junction 316B, and second electrically conductive material 302 are furthermore flush with an outer surface 354 of the matrix material 350.

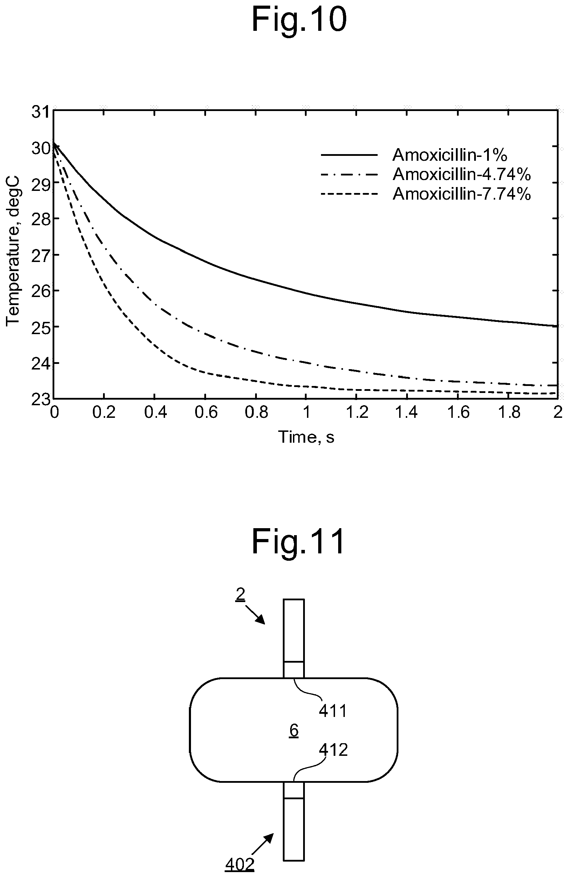

Figures 9 and 10 are graphs depicting data obtained using an apparatus 2 of the type depicted in Figure 1 to measure the variation with time of a local temperature of a sample after application of a heating pulse via Joule heating applied through the second conductor 302. Four curves are shown in Figure 9, respectively showing measured variation of the temperature of air, oil, amoxicillin and water. The four curves are highly distinct, even between the three liquid samples, demonstrating that this simple technique is capable of distinguishing with high sensitivity between different materials. Figure 10 compares three samples having different concentrations of the antibiotic amoxicillin. Again, the three different concentrations lead to three clearly distinguishable thermal responses that can be used to distinguish between the different samples. Figure 10 demonstrates how the approach can be used specifically to detect dilution of medicines such as antibiotics.

Figure 11 depicts a further embodiment where the thermal response is measured at multiple points around a solid sample 6. In embodiments of this type a thermal response of the sample 6 to the heating is measured with the sensor element 2 in contact (e.g. in direct contact with a surface of the sample or in contact via a thin coupling layer or gel not forming an integral part of the sample 6) with a first region 411 on the surface of the sample 6 and a further thermal response of the sample 6 to the heating is measured using a further sensor element 402 in contact (e.g. in direct contact with a surface of the sample or in contact via a thin coupling layer or gel not forming an integral part of the sample 6) with a second region 412 on the surface of the sample 6. The second region 412 is separate (i.e. not in contact with or overlapping with) the first region 411. The further sensor element 402 may take any of the forms described above for the sensor element 2. Alternatively, the further sensor element 402 may have a simpler construction, being configured for example only to measure temperature (rather than being able to provide heating and measure temperature, as is possible in embodiments of the sensor element 2). The further sensor element 402 may thus comprise a thermocouple device of the type discussed above with reference to Figure 3 for example. The provision of the further sensor element 402 makes it possible to measure a thermal response of the sample 6 to the heating provided by the sensor element 2 at a distance which is further away from the sensor element 2. The heat can thus be made to propagate through a large proportion of the sample 6 before reaching the further sensor element 402. This approach can enhance the ability of the method to sample properties of the sample 6 deep within the sample 6 and/or to measure thicker sample effectively. In an embodiment, the second region 412 is on an opposite side of the sample 6 to the first region 411, which provides a particularly efficient way of sampling properties of the sample 6 deep within the sample 6.

**Claims**

1. An apparatus (2) for testing a sample (6), comprising: a sensor element (4) comprising a first conductor, a second conductor and a third conductor, wherein:

   the first conductor (301), second conductor (302) and third conductor (303) are connected together electrically in series;
   the first conductor (301) is connected to the second conductor (302) at a first sensing junction (316A);
   the second conductor (302) is connected to the third conductor (303) at a second sensing junction (316B); and
   the apparatus (2) further comprises a measurement unit (12), **characterized in that** the measurement unit (12) is configured to:

   apply heating to a sample (6) under test via Joule heating in the second conductor (302), the Joule heating being generated by driving an electrical current in series through the first conductor (301), second conductor (302) and third conductor (303);
   measure a thermal response of the sample (6) to the heating by measuring a potential difference between the first conductor (301) and the third conductor (303), wherein the potential difference is influenced by a temperature gradient along the first conductor (301) and a temperature gradient along the third conductor (303) via the Seebeck effect; and
   generate an indication of whether the sample (6) satisfies a predetermined criterion based on the measured thermal response.

2. The apparatus (2) of claim 1, wherein a majority of the electrical resistance in the circuit through which current is driven to provide the Joule heating is contributed by the second conductor (302).

3. The apparatus (2) of claim 1 or 2, wherein the electrical resistance of the second conductor (302) is at least 2 times higher than the electrical resistance of the first conductor (301) and than the electrical resistance of the third conductor (303).

4. The apparatus (2) of any preceding claim, wherein the second conductor (302) is locally elongate and has a length that is at least 2 times longer than a shortest distance between the first sensing junction (316A) and the second sensing junction (316B), wherein the second conductor (302) optionally comprises a spiral, a helix or a serpentine shape.

5. The apparatus (2) of any preceding claim, wherein an average cross-sectional area of the second conductor (302) is at least 2 times smaller than an average cross-sectional area of the first conductor (301) and than an average cross-sectional area of the third conductor (303).

6. The apparatus (2) of any preceding claim, wherein the first sensing junction (316A) is formed by welding of the first conductor (301) to the second conductor (302) and the second sensing junction (316B) is formed by welding of the second conductor (302) to the third conductor (303).

7. The apparatus (2) of any preceding claim, wherein the first conductor (301), second conductor (302) and third conductor (303) all have different compositions, the first conductor (301) and third conductor (303) optionally being respectively formed from one of the following pairs of materials:

   chromel and constantan as for a type E thermocouple device;
   iron and constantan as for a type J thermocouple device;
   chromel and alumel as for a type K thermocouple device;
   82%Ni/18%Mo and 99.2%Ni/0.8%Co, by weight, as for a type M thermocouple device;
   Nicrosil and Nisil as for a type N thermocouple device;
   copper and constantan as for a type T thermocouple;
   70%Pt/30%Rh and 94%Pt/6%Rh, by weight, as for a type B thermocouple device;
   87%Pt/13%Rh by weight and platinum as for a type R thermocouple device;
   90%Pt/10%Rh by weight and platinum as for a type S thermocouple device;
   95%W/5%Re and 74%W/26%Re, by weight, as for a type C thermocouple device;
   97%W/3%Re and 75%W/25%Re, by weight, as for a type D thermocouple device; and
   Tungsten and 74%W/26%Re by weight as for a type G thermocouple device.

8. The apparatus (2) of any preceding claim, wherein:

   each of at least part of the first conductor (301), at least part of the second conductor (302), and at least part of the third conductor (303) is embedded in a matrix material; and one or more of the following are flush with an outer surface of the matrix material and can be brought into direct contact with the sample (6) : the first sensing junction (316A), the second sensing junction (316B), and the second conductor (302).

9. The apparatus (2) of any preceding claim, wherein the heating of the sample (6) and the measurement of the thermal response are performed in non-overlapping time periods.

10. A method of testing a sample (6), comprising: providing a sensor element (4) comprising a first conductor (301), a second conductor (302) and a third conductor (303), wherein:

    the first conductor (301), second conductor (302) and third conductor (303) are connected together electrically in series; the first conductor (301) is connected to the second conductor (302) at a first sensing junction (316A); the second conductor (302) is connected to the third conductor (303) at a second sensing junction (316B); and **characterized in that** the method further comprises:

    applying heating to a sample (6) under test via Joule heating in the second conductor (302), the Joule heating being generated by driving an electrical current in series through the first conductor (301), second conductor (302) and third conductor (303); measuring a thermal response of the sample (6) to the heating by measuring a potential difference between the first conductor (301) and the third conductor (303), wherein the potential difference is influenced by a temperature gradient along the first conductor (301) and a temperature gradient along the third conductor (303) via the Seebeck effect; and generating an indication of whether the sample (6) satisfies a predetermined criterion based on the measured thermal response.

11. The method claim 10, the heating of the sample (6) and the measurement of the thermal response are performed in non-overlapping time periods.

12. The method of claim 10 or 11, or the apparatus (2) of any of claims 1-9, wherein the sample (6) comprises a pharmaceutical product.

13. The method of any of claims 10-12, or the apparatus of any of claims 1-9 or 12, wherein the sample (6) comprises a liquid.

14. The method of any of claims 10-12, or the apparatus of any of claims 1-9 or 12, wherein the sample (6) comprises a solid body.

15. The method of claim 14, wherein:

    the thermal response of the sample (6) to the heating is measured with the sensor element (4) in contact with a first region (411) on the sample (6); and a further thermal response of the sample (6) to the heating is measured using a further sensor element (402) in contact with a second region (412) on the sample (6), the second region (412) being separate from the first region (411) and optionally on an opposite side of the sample (6) to the first region (411).

**Patentansprüche**

1. Vorrichtung (2) zum Testen einer Probe (6), umfassend: ein Sensorelement (4), umfassend einen ersten Leiter, einen zweiten Leiter und einen dritten Leiter, wobei:

   der erste Leiter (301), zweite Leiter (302) und dritte Leiter (303) zusammen elektrisch in Reihe verbunden sind; der erste Leiter (301) mit dem zweiten Leiter (302) an einer ersten Sensorverbindung (316A) verbunden ist; der zweite Leiter (302) mit dem dritten Leiter (303) an einer zweiten Sensorverbindung (316B) verbunden ist; und die Vorrichtung (2) ferner eine Messeinheit (12) umfasst, **dadurch gekennzeichnet, dass** die Messeinheit (12) für Folgendes konfiguriert ist:

   Aufbringen von Wärme auf eine zu prüfende Probe (6) über Joule-Erwärmung in dem zweiten Leiter (302), wobei die Joule-Erwärmung durch Antreiben eines elektrischen Stroms in Reihe durch den ersten Leiter (301), zweiten Leiter (302) und dritten Leiter (303) erzeugt wird; Messen einer thermischen Reaktion der Probe (6) auf das Erwärmen durch Messen einer Potentialdifferenz zwischen dem ersten Leiter (301) und dem dritten Leiter (303), wobei die Potentialdifferenz durch einen Temperaturgradienten entlang des ersten Leiters (301) und einen Temperaturgradienten entlang des dritten Leiters (303) über den Seebeck-Effekt beeinflusst wird; und Erzeugen einer Angabe darüber, ob die Probe (6) ein vorbestimmtes Kriterium erfüllt, basierend auf der gemessenen thermischen Reaktion.

2. Vorrichtung (2) nach Anspruch 1, wobei ein Großteil des elektrischen Widerstands in dem Stromkreis, durch den Strom fließt, um die Joule-Erwärmung bereitzustellen, durch den zweiten Leiter (302) beigetragen wird.

3. Vorrichtung (2) nach Anspruch 1 oder 2, wobei der elektrische Widerstand des zweiten Leiters (302) mindestens 2-mal größer als der elektrische Widerstand des ersten Leiters (301) und als der elektrische Widerstand des dritten Leiters (303) ist.

4. Vorrichtung (2) nach einem vorhergehenden Anspruch, wobei der zweite Leiter (302) lokal länglich ist und eine Länge hat, die mindestens 2-mal länger als ein kürzester Abstand zwischen der ersten Sensorverbindung (316A) und der zweiten Sensorverbindung (316B) ist, wobei der zweite Leiter (302) optional eine Spiral- Helix- oder Serpentinenform umfasst.

5. Vorrichtung (2) nach einem vorhergehenden Anspruch, wobei eine durchschnittliche Querschnittsfläche des zweiten Leiters (302) mindestens 2-mal kleiner als eine durchschnittliche Querschnittsfläche des ersten Leiters (301) und als eine durchschnittliche Querschnittsfläche des dritten Leiters (303) ist.

6. Vorrichtung (2) nach einem vorhergehenden Anspruch, wobei die erste Sensorverbindung (316A) durch Schweißen des ersten Leiters (301) an den zweiten Leiter (302) gebildet ist und die zweite Sensorverbindung (316B) durch Schweißen des zweiten Leiters (302) an den dritten Leiter (303) gebildet ist.

7. Vorrichtung (2) nach einem vorhergehenden Anspruch, wobei der erste Leiter (301), zweite Leiter (302) und dritte Leiter (303) verschiedene Zusammensetzungen haben, wobei der erste Leiter (301) und dritte Leiter (303) optional entsprechend aus einem der folgenden Materialpaare gebildet sind:

Chromel und Konstantan wie für eine Typ-E-Thermoelementvorrichtung;
Eisen und Konstantan wie für eine Typ-J-Thermoelementvorrichtung;
Chromel und Alumel wie für eine Typ-K-Thermoelementvorrichtung;
82 Gew.-% Ni/18 Gew.-% Mo und 99,2 Gew.-% Ni/0,8 Gew.-% Co wie für eine Typ-M-Thermoelementvorrichtung;
Nicrosil und Nisil wie für eine Typ-N-Thermoelementvorrichtung;
Kupfer und Konstantan wie für ein Typ-T-Thermoelement;
70 Gew.-% Pt/30 Gew.-% Rh und 94 Gew.-% Pt/6 Gew.-% Rh wie für eine Typ-B-Thermoelementvorrichtung;
87 Gew.-% Pt/13 Gew.-% Rh und Platin wie für eine Typ-R-Thermoelementvorrichtung;
90 Gew.-% Pt/10 Gew.-% Rh und Platin wie für eine Typ-S-Thermoelementvorrichtung;
95 Gew.-% W/5 Gew.-% Re und 74 Gew.-% W/26 Gew.-% Re wie für eine Typ-C-Thermoelementvorrichtung;
97 Gew.-% W/3 Gew.-% Re und 75 Gew.-% W/25 Gew.-% Re wie für eine Typ-D-Thermoelementvorrichtung; und
Wolfram und 74 Gew.-% W/26 Gew.-% Re wie für eine Typ-G-Thermoelementvorrichtung.

8. Vorrichtung (2) nach einem vorhergehenden Anspruch, wobei:

jeder aus mindestens einem Teil des ersten Leiters (301), mindestens einem Teil des zweiten Leiters (302) und mindestens einem Teil des dritten Leiters (303) in einem Matrixmaterial eingebettet ist; und
eines oder mehrere des Folgenden bündig mit einer Außenfläche des Matrixmaterials sind und in direkten Kontakt mit der Probe (6) gebracht werden können: die erste Sensorverbindung (316A), die zweite Sensorverbindung (316B) und der zweite Leiter (302).

9. Vorrichtung (2) nach einem vorhergehenden Anspruch, wobei das Erwärmen der Probe (6) und die Messung der thermischen Reaktion in nicht-überlagernden Zeiträumen durchgeführt werden.

10. Verfahren des Testens einer Probe (6), umfassend: Bereitstellen eines Sensorelements (4), umfassend einen ersten Leiter (301), einen zweiten Leiter (302) und einen dritten Leiter (303), wobei:

der erste Leiter (301), zweite Leiter (302) und dritte Leiter (303) zusammen elektrisch in Reihe verbunden sind;
der erste Leiter (301) mit dem zweiten Leiter (302) an einer ersten Sensorverbindung (316A) verbunden ist;
der zweite Leiter (302) mit dem dritten Leiter (303) an einer zweiten Sensorverbindung (316B) verbunden ist; und

**dadurch gekennzeichnet, dass** das Verfahren ferner Folgendes umfasst:

Aufbringen von Wärme auf eine zu prüfende Probe (6) über Joule-Erwärmung in dem zweiten Leiter (302), wobei die Joule-Erwärmung durch Antreiben eines elektrischen Stroms in Reihe durch den ersten Leiter (301), zweiten Leiter (302) und dritten Leiter (303) erzeugt wird;

Messen einer thermischen Reaktion der Probe (6) auf das Erwärmen durch Messen einer Potentialdifferenz zwischen dem ersten Leiter (301) und dem dritten Leiter (303), wobei die Potentialdifferenz durch einen Temperaturgradienten entlang des ersten Leiters (301) und einen Temperaturgradienten entlang des dritten Leiters (303) über den Seebeck-Effekt beeinflusst wird; und

Erzeugen einer Angabe darüber, ob die Probe (6) ein vorbestimmtes Kriterium erfüllt, basierend auf der gemessenen thermischen Reaktion.

11. Verfahren nach Anspruch 10, wobei das Erwärmen der Probe (6) und die Messung der thermischen Reaktion in nicht-überlagernden Zeiträumen durchgeführt werden.

12. Verfahren nach Anspruch 10 oder 11 oder Vorrichtung (2) nach einem der Ansprüche 1-9, wobei die Probe (6) ein pharmazeutisches Produkt umfasst.

13. Verfahren nach einem der Ansprüche 10-12 oder Vorrichtung nach einem der Ansprüche 1-9 oder 12, wobei die Probe (6) eine Flüssigkeit ist.

14. Verfahren nach einem der Ansprüche 10-12 oder Vorrichtung nach einem der Ansprüche 1-9 oder 12, wobei die Probe (6) einen Festkörper umfasst.

15. Verfahren nach Anspruch 14, wobei:

die thermische Reaktion der Probe (6) auf das Erwärmen mit dem Sensorelement (4) in Kontakt mit einem ersten Bereich (411) auf der Probe (6) gemessen wird; und

eine weitere thermische Reaktion der Probe (6) auf das Erwärmen unter Verwendung eines weiteren Sensorelements (402) in Kontakt mit einem zweiten Bereich (412) auf der Probe (6) gemessen wird, wobei der zweite Bereich (412) getrennt von dem ersten Bereich (411) und optional auf einer gegenüberliegenden Seite der Probe (6) zu dem ersten Bereich (411) ist.

**Revendications**

1. Appareil (2) pour tester un échantillon (6), comprenant :

un élément de détection (4) comprenant un premier conducteur, un deuxième conducteur et un troisième conducteur ; étant entendu que

le premier conducteur (301), le deuxième conducteur (302) et le troisième conducteur (303) sont connectés ensemble électriquement en série ;

le premier conducteur (301) est connecté au deuxième conducteur (302) au niveau d'une première jonction de détection (316A) ;

le deuxième conducteur (302) est connecté au troisième conducteur (303) au niveau d'une deuxième jonction de détection (316B) ; et

l'appareil (2) comprend en outre une unité de mesure (12) ; **caractérisé en ce que** l'unité de mesure (12) est configurée pour :

chauffer un échantillon (6) soumis à un test via un chauffage par effet Joule dans le deuxième conducteur (302), le chauffage par effet Joule étant généré en faisant passer un courant électrique en série à travers le premier conducteur (301), le deuxième conducteur (302) et le troisième conducteur (303) ;

mesurer une réponse thermique de l'échantillon (6) au chauffage en mesurant une différence de potentiel entre le premier conducteur (301) et le troisième conducteur (303), la différence de potentiel étant influencée par un gradient de température le long du premier conducteur (301) et un gradient de température le long du troisième conducteur (303) via l'effet Seebeck ; et

générer une indication indiquant si l'échantillon (6) répond à un critère prédéterminé compte tenu de la réponse thermique mesurée.

2. Appareil (2) selon la revendication 1, dans lequel une majorité de la résistance électrique dans le circuit à travers lequel le courant est conduit pour fournir le chauffage par effet Joule est apportée par le deuxième conducteur (302).

3. Appareil (2) selon la revendication 1 ou 2, dans lequel la résistance électrique du deuxième conducteur (302) est au moins deux fois supérieure à la résistance électrique du premier conducteur (301) et à la résistance électrique du troisième conducteur (303).

4. Appareil (2) selon l'une quelconque des revendications précédentes, dans lequel le deuxième conducteur (302) est localement allongé et a une longueur qui est au moins deux fois supérieure à la distance la plus courte entre la première jonction de détection (316A) et la deuxième jonction de détection (316B), ledit deuxième conducteur (302) comprenant éventuellement une forme en spirale, en hélice ou en serpentin.

5. Appareil (2) selon l'une quelconque des revendications précédentes, dans lequel la section transversale moyenne du deuxième conducteur (302) est au moins deux fois plus petite que la section transversale moyenne du premier conducteur (301) et que la section transversale moyenne du troisième conducteur (303).

6. Appareil (2) selon l'une quelconque des revendications précédentes, dans lequel la première jonction de détection (316A) est formée par soudage du premier conducteur (301) au deuxième conducteur (302) et la deuxième jonction de détection (316B) est formée par soudage du deuxième conducteur (302) au troisième conducteur (303).

7. Appareil (2) selon l'une quelconque des revendications précédentes, dans lequel le premier conducteur (301), le deuxième conducteur (302) et le troisième conducteur (303) ont tous des compositions différentes, le premier conducteur (301) et le troisième conducteur (303) étant éventuellement respectivement formés à partir de l'une des paires de matériaux suivantes :

chromel et constantan comme pour un dispositif thermocouple de type E ;
fer et constantan comme pour un dispositif thermocouple de type J ;
chromel et alumel comme pour un dispositif thermocouple de type K ;
82 % de Ni / 18 % de Mo et 99,2 % de Ni / 0,8 % de Co, en poids, comme pour un dispositif thermocouple de type M ;
nicrosil et nisil comme pour un dispositif thermocouple de type N ;
cuivre et constantan comme pour un thermocouple de type T ;
70 % de Pt / 30 % de Rh et 94 % de Pt / 6 % de Rh, en poids, comme pour un dispositif thermocouple de type B ;
87 % de Pt/13% de Rh en poids et platine comme pour un dispositif thermocouple de type R ;
90 % de Pt/10% de Rh en poids et platine comme pour un dispositif thermocouple de type S ;
95 % de W / 5 % de Re et 74 % de W / 26 % de Re, en poids, comme pour un dispositif thermocouple de type C ;
97 % de W / 3 % de Re et 75 % de W / 25 % de Re, en poids, comme pour un dispositif thermocouple de type D ; et
tungstène et 74 % de W / 26 % de Re en poids comme pour un dispositif thermocouple de type G.

8. Appareil (2) selon l'une quelconque des revendications précédentes, dans lequel :

au moins une partie du premier conducteur (301), au moins une partie du deuxième conducteur (302) et au moins une partie du troisième conducteur (303) sont respectivement encastrées dans un matériau de matrice ; et
un ou plusieurs des éléments suivants sont de niveau avec une surface externe du matériau de matrice et peuvent être mis en contact direct avec l'échantillon (6) : la première jonction de détection (316A), la deuxième jonction de détection (316B) et le deuxième conducteur (302).

9. Appareil (2) selon l'une quelconque des revendications précédentes, dans lequel le chauffage de l'échantillon (6) et la mesure de la réponse thermique sont effectués pendant des périodes non concomitantes.

10. Procédé de test d'un échantillon (6), comprenant : la fourniture d'un élément de détection (4) comprenant un premier conducteur (301), un deuxième conducteur (302) et un troisième conducteur (303) ; étant entendu que :

le premier conducteur (301), le deuxième conducteur (302) et le troisième conducteur (303) sont connectés ensemble électriquement en série ;
le premier conducteur (301) est connecté au deuxième conducteur (302) au niveau d'une première jonction de détection (316A) ;
le deuxième conducteur (302) est connecté au troisième conducteur (303) au niveau d'une deuxième jonction de détection (316B) ; et
le procédé étant **caractérisé en ce qu'**il comprend en outre :

le chauffage d'un échantillon (6) soumis à un test via un chauffage par effet Joule dans le deuxième conducteur (302), le chauffage par effet Joule étant généré par le passage d'un courant électrique en série à travers le premier conducteur (301), le deuxième conducteur (302) et le troisième conducteur (303) ;
la mesure d'une réponse thermique de l'échantillon (6) au chauffage par la mesure d'une différence de potentiel entre le premier conducteur (301) et le troisième conducteur (303), la différence de potentiel étant influencée par un gradient de température le long du premier conducteur (301) et un gradient de température le long du troisième conducteur (303) via l'effet Seebeck ; et

la génération d'une indication indiquant si l'échantillon (6) répond à un critère prédéterminé compte tenu de la réponse thermique mesurée.

11. Procédé selon la revendication 10, le chauffage de l'échantillon (6) et la mesure de la réponse thermique sont effectués pendant des périodes non concomitantes.

12. Procédé selon la revendication 10 ou 11, ou appareil (2) selon l'une quelconque des revendications 1 à 9, dans lequel l'échantillon (6) comprend un produit pharmaceutique.

13. Procédé selon l'une quelconque des revendications 10 à 12, ou appareil selon l'une quelconque des revendications 1 à 9 ou 12, dans lequel l'échantillon (6) comprend un liquide.

14. Procédé selon l'une quelconque des revendications 10 à 12, ou appareil selon l'une quelconque des revendications 1 à 9 ou 12, dans lequel l'échantillon (6) comprend un corps solide.

15. Procédé selon la revendication 14, dans lequel :

la réponse thermique de l'échantillon (6) au chauffage est mesurée à l'aide de l'élément de détection (4) en contact avec une première région (411) sur l'échantillon (6) ; et une autre réponse thermique de l'échantillon (6) au chauffage est mesurée à l'aide d'un autre élément capteur (402) en contact avec une deuxième région (412) sur l'échantillon (6), la deuxième région (412) étant séparée de la première région (411) et éventuellement sur un côté de l'échantillon (6) opposé à la première région (411).

# Fig.1

# Fig.2

# Fig.3

# Fig.4

# Fig.5

# Fig.6

# Fig.7

# Fig.8

# Fig.9

# Fig.10

# Fig.11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107271481 A
- RU 2273005 C1